# EUROPÄISCHE PATENTSCHRIFT

(11) **EP 3 816 172 B1**
(45) Veröffentlichungstag und Bekanntmachung des Hinweises auf die Patenterteilung: **22.12.2021**
(21) Anmeldenummer: 19205651.3
(22) Anmeldetag: 28.10.2019
(51) Int. Cl.: C07F 9/6574, B01J 31/18, C07B 41/06, C07C 45/50, C07F 15/00

(54) **PHOSPHAZYKLISCHE PHOSPHITE AUS DEM ENOL DES 1-HYDROXY-2-ACETONAPHTHONS**
PHOSPHACYCLIC PHOSPHITES FROM THE ENOL 1-HYDROXY-2-ACETONAPHTHONE
PHOSPHITES PHOSPHOCYCLIQUES DE L'ÉNOL DU 1-HYDROXY-2-ACÉTONAPHTHONE

(43) Veröffentlichungstag der Anmeldung: 05.05.2021
(73) Patentinhaber: Evonik Operations GmbH, 45128 Essen (DE)
(72) Erfinder: FRANKE, Robert, 45772 Marl (DE); BÖRNER, Armin, 18059 Rostock (DE); SELENT, Detlef, 18059 Rostock (DE)
(74) Vertreter: Evonik Patent Association

(56) Entgegenhaltungen:
- EP-A1- 1 201 675
- EP-A1- 2 003 138

## Beschreibung

Die Erfindung betrifft phosphazyklische Phosphite aus dem Enol des 1-Hydroxy-2-acetonaphthons.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

In EP 2003138A1 wird ein Verfahren zur Herstellung von Aldehyden beschrieben. Eine Kohlenstoff-Kohlenstoff-Doppelbindung wird mit Kohlenmonoxid und Wasserstoff in Gegenwart eines Bisphosphits und einer Metallverbindung der Gruppen 8 bis 10 umgesetzt. EP 1 201 675 A1 offenbart Acylphosphite der Formel (I) und ihre Verwendung zur Katalyse einer Hydroformylierungsreaktion.

Das Hydroxyketon des 1-Hydroxy-2-acetonaphthons weist ein Keto-Enol-Gleichgewicht auf:

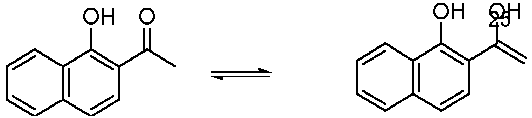

Die technische Aufgabe der Erfindung ist die Bereitstellung neuer Liganden, welche in der Hydroformylierung von Olefinen eine gesteigerte Ausbeute aufweisen.

Die Aufgabe wird gelöst durch eine Verbindung gemäß Anspruch 1.

Verbindung gemäß der Struktur (**I**): 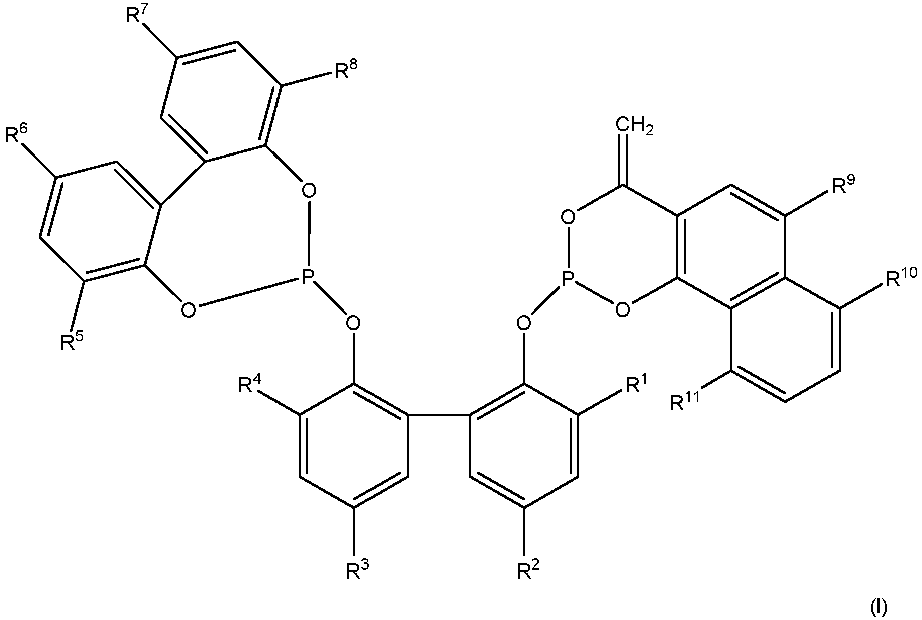 wobei R¹, R², R³, R⁴, R⁵, R⁶, R⁷, R⁸, R⁹, R¹⁰, R¹¹ ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -NO₂, -NH₂, -N[(C₁-C₁₂)-Alkyl]₂.

In einer Ausführungsform sind R¹, R², R³, R⁴ ausgewählt aus: -H, -(C₁-C₁₂)-Alkyl, -O-(C₁-C₁₂)-Alkyl.

In einer Ausführungsform sind R⁵, R⁶, R⁷, R⁸ ausgewählt aus: -H, -(C₁-C₁₂)-Alkyl, -O-(C₁-C₁₂)-Alkyl.

In einer Ausführungsform sind R⁹, R¹⁰, R¹¹ ausgewählt aus: -H, -(C₁-C₁₂)-Alkyl, -O-(C₁-C₁₂)-Alkyl.

In einer Ausführungsform sind R¹, R², R³, R⁴, R⁵, R⁶, R⁷, R⁸, R⁹, R¹⁰, R¹¹ ausgewählt aus: -H, - (C₁-C₁₂)-Alkyl, -O-(C₁-C₁₂)-Alkyl.

In einer Ausführungsform weist die Verbindung die Struktur (1) auf:

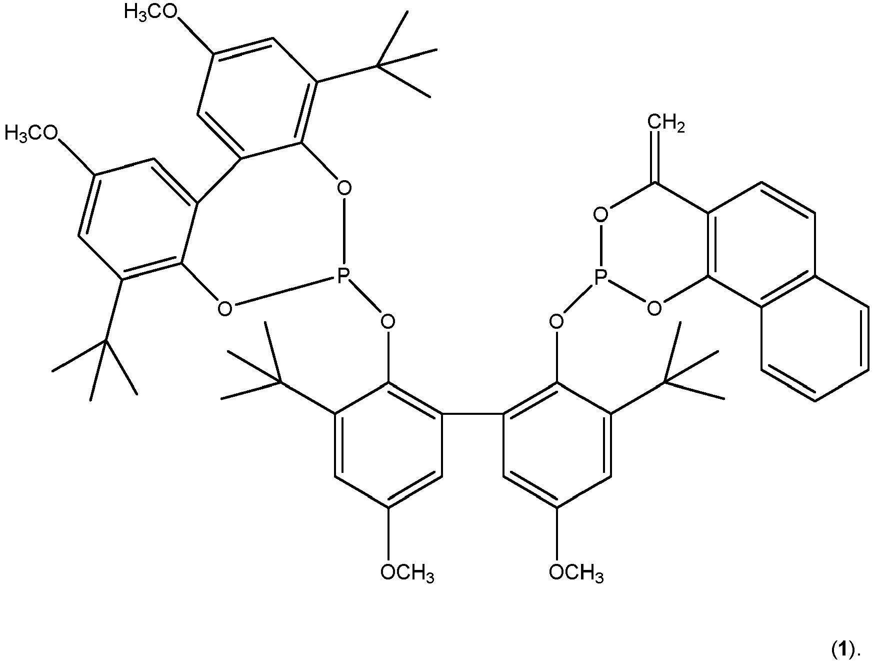

Neben der Verbindung als solche wird auch deren Verwendung zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird ein Verfahren beansprucht, in welchem die zuvor beschriebene Verbindung als Ligand eingesetzt wird.

Verfahren umfassend die Verfahrensschritte:
a) Vorlegen eines Olefins,
b) Zugabe einer zuvor beschriebenen Verbindung und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

In einer bevorzugten Ausführungsform ist das Metall Rh. Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 5 bis 70 bar. Besonders bevorzugt sind eine Temperatur von 100 °C bis 140 °C und ein Druck von 5 bis 50 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei derTetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden α-Olefine, endständig verzweigte, innenständige und innenständig verzweigte Olefine hydroformyliert werden.

Im Folgenden soll die Erfindung anhand von Ausführungsbeispielen näher erläutert werden.

### Arbeitsvorschriften

### Allgemeine Analytik

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet.

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR³¹P = SR¹H * (BF³¹P / BF¹H) = SR¹H * 0,4048.

### Synthese 2-Chlor-4-methylen-4H-naphtho[1,2-d][1,3,2]dioxaphosphinin (Intermediat A)

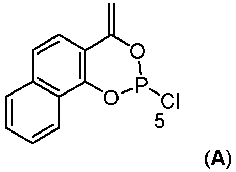

Zu einer Lösung von Phosphortrichlorid (5,23 ml; 60 mmol) in THF (25 ml) wird bei -20°C starkem Rühren langsam eine Mischung von 1-Hydroxy-2-acetonaphthon (2,235 g; 12,0 mmol) und Pyridin (3,9 ml) in THF (25 ml) getropft. Man lässt auf Raumtemperatur kommen, rührt über Nacht und filtriert. Das Filtrat wird im Vakuum zur Trockne eingeengt und 4 h bei 50° C/0,1 mbar getrocknet. Ausbeute: 2,774 g (11,06 mmol; 92 %). Das so erhaltene Produkt wurde ohne weitere Aufreinigung eingesetzt.
³¹P-NMR (CD₂Cl₂): 133,9 (s) ppm.
¹H-NMR (CD₂Cl₂): 5,01 (m, 1H); 5,41 (s, 1H); 7,62 (m, 4H); 7,86 (m, 1H); 8,19 (m, 1H) ppm.

### Synthese 4,8-Di-tert.-butyl-6-((3,3'-di-tert.-butyl-2'-((dichlorophosphanyl)oxy)-5,5'-dimethoxy-[1,1'-biphenyl]-2-yl)oxy)-2.10-dimethoxydibenzo[d,f][1,3,2]dioxaphosphepin (Intermediat B)

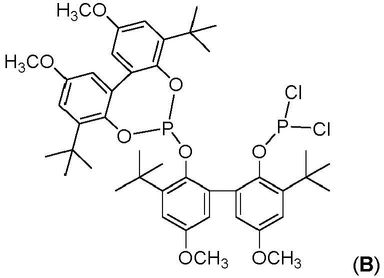

Zu einer Mischung aus 3,3'-Di-tert.-butyl-2'-((4,8-di-tert.-butyl-2,10-dimethoxy-dibenzo[*d*,*f*][1,3,2]dioxaphosphepin-6-yl)oxy)-5,5'-dimethoxy-[1,1'-biphenyl]-2-ol (4,925 g; 6,61 mmol, hergestellt nach D. Selent, D. Hess, K.-D. Wiese, D. Röttger, C. Kunze, A. Börner, Angew. Chem. 2001, 113, 1739) und Triethylamin (1,83 ml) in Toluol (60 ml) wird bei 0°C unter Rühren eine Lösung von Phosphortrichlorid (1,089 g; 7,93 mmol) in Toluol (15 ml) getropft. Man rührt anschließend bei Raumtemperatur über Nacht und zusätzlich 3 h bei 85°C Badtemperatur. Die nach Filtration erhaltene Lösung wird zur Trockne eingeengt. Der erhaltene Rückstand wird 3 h bei 60 °C/0,1 mbar getrocknet und dann in Hexan (70 ml) verrührt. Man lässt die Mischung 3 h bei 5°C stehen und filtriert bei 0°C. Der erhaltene Feststoff wird mit gekühltem Hexan (1x15 ml) gewaschen und 3 h bei 45 °C / 0,1 mbar getrocknet. Das Filtrat der letzten Filtration wird auf 1/3 des Volumens eingeengt, 3 Tage bei 5 °C gelagert und der ausgefallene Feststoff wie oben beschrieben behandelt. Gesamtausbeute: 4,95 g (5,85 mmol; 88%).
³¹P-NMR (CD₂Cl₂): 141,5 (s); 203,8 (s) ppm.
EI-MS (70 eV): *m*/*z*=844 [M⁺].

### Synthese 4,8-Di-tert-butyl-6-((3,3'-di-tert-butyl-5,5'-dimethoxy-2'-((4-methylen-4H-naphtho[1,2-d][1,3,2]dioxaphosphinin-2-yl)oxy)-[1,1'-biphenyl]-2-yl)oxy)-2,10-dimethoxy-dibenzo[d,f][1,3,2]dioxaphosphepin (1)

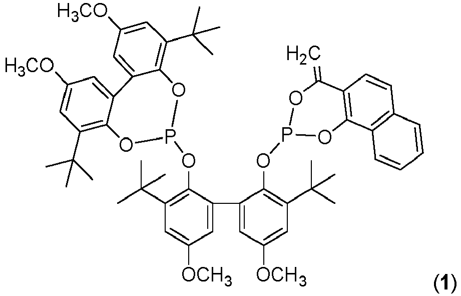

Eine Lösung des Intermediates B (0,803 g; 0,949 mmol) in Toluol (8 ml) wird bei Raumtemperatur mit einer Mischung von 1-Hydroxy-2-acetonaphthon (0,177 g; 0,949 mmol) und Triethylamin (0,53 ml) in Toluol (5 ml) versetzt. Man lässt über Nacht bei Raumtemperatur rühren, filtriert, engt das Filtrat im Vakuum ein und trocknet den weißen Rückstand wird zunächst 4 h bei 50 °C / 0,1 mbar. Die säulenchromatografische Reinigung (Dichlormethan/Hexan=1:4, R*_{f}*=0,56) ergibt 0,708 g (0,738 mmol; 78%).

Elementaranalyse (berechnet für C₅₆H₆₄O₁₀P₂=959,06 g/mol): C=70,36 (70,13); H=6,59 (6,46); P=6,25 (6,56) %.
³¹P-NMR (CD₂Cl₂): 107,7 (d, Jpp= 39,2 Hz); 109,9 (d, *J*_{PP}= 25,7 Hz); 138,1-140,3 ppm.
¹H-NMR (CD₂Cl₂): 0,96-1,29 (überlappende Signale, ^{t}Bu, 36H); 3,61-3,72 (überlappende Signale, O-CH₃, 12H), 4,35+4,53; 4,93+5,03; 6,50-7,97 (m, Harom, 14H) ppm.
ESI-TOF HRMS: *m*/*z*=981,3870, [M⁺+Na], berechnet *m*/*z*=981,3872.

### Synthese 2,2'-((3.3'-Di-tert-butyl-5,5'-dimethoxy-[1,1'-biphenyl]-2.2'-diyl)bis(4-methylen-4H-naphtho[1,2-d][1,3,2]dioxaphosphinin) (2) (Vergleichsligand)

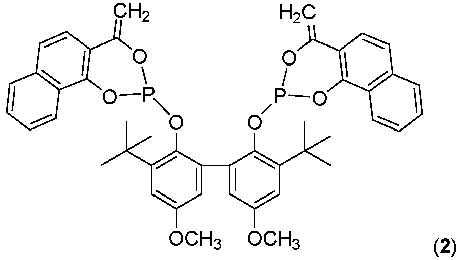

Zu einer auf 0°C gekühlten Lösung von Intermediat **A** (1,111 g; 4,433 mmol) in THF (15 ml) wird tropfenweise eine Mischung aus 3,3'-Di-tert.-butyl-5,5'-dimethoxy-[1,1'-biphenyl]-2,2'-diol (0,756 g; 2,110 mmol) und Pyridin (0,4 ml) in THF (8 ml) gegeben. Man lässt auf Raumtemperatur kommen, rührt über Nacht und filtriert. Das Filtrat wird im Vakuum vom Lösungsmittel befreit, der erhaltene hellgelbe Feststoff 4 h bei 50° C/0,1 mbar getrocknet und dann säulenchromatografisch (Dichlormethan, R*_{f}*= 0,74) gereinigt. Ausbeute: 0,470 g (0,597 mmol; 28%).
Elementaranalyse (berechnet für C₄₄H₄₄O₈P₂=786,80 g/mol): C=70,34 (70,22); H=5,50 (5,64) %. ³¹P-NMR (CD₂Cl₂): 105,1; 106,4; 106,9; 107,8 ppm.
¹H-NMR (CD₂Cl₂): 1,12 (s, 4,5 H); 1,13 (s, 4,5 H); 1,30 (s, 4,5 H); 1,37 (s, 4,5 H); 3,60 (s, 1,5 H); 3,69 (s, 1,5 H); 3,82 (s, 1,5 H), 3,85 (s, 1,5 H); 4,50-4,66 (4 m, 2H); 5,11-5,17 (2 m, 2 H); 6,59-6,72 (3 m, 2 H); 6,99-7,09 (2 m, 2 H); 7,53-7,59 (m, 8 H); 7,78-7,86 (2 m, 2 H); 7,91-8,20 (3 m, 2 H) ppm. ESI-TOF HRMS: m/z=787,2585 [M⁺+H], berechnet m/z=787,2590.

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzten Olefin cis/trans-2-Penten (Aldrich) wurden über Natrium am Rückfluss erhitzt und unter Argon destilliert. Im Autoklaven wurden unter Argonatmosphäre Lösungen der Katalysatorvorstufe und des Liganden, jeweils in Toluol, gemischt. Als Katalysatorvorstufe kam [(acac)Rh(COD)] (Umicore, acac=Acetylacetonat-Anion; COD=1,5-Cyclooctadien), zum Einsatz. Der Autoklav wurde bei einem Gesamtgasdruck (Synthesegas: Linde; H2 (99,999%) : CO (99,997%) = 1:1) von 4 bar für den Enddruck von 10 bar unter Rühren (1500 U/min) aufgeheizt. Nach Erreichen der Reaktionstemperatur, 120 °C, wurde das Olefin mit einem in der Druckpipette eingestellten Überdruck in den Autoklaven gepresst. Die Reaktion wurde bei konstantem Druck, 10 bar (Nachdruckregler der Fa. Bronkhorst, NL), über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m × 0,2 mm × 0,5 µm.

### Ergebnisse der Katalyseversuche

[Rh]: 100 ppm, Rh:L = 1:2, p: 10 bar, T: 120 °C; t: 4 h

**Tabelle 1: Hydroformylierung 2-Penten**

| Eintrag | Ligand | Ausbeute in % |
|---|---|---|
| 1 | **1*** | 96 |
| 2 | **2** | 83 |

| | | |
|---|---|---|
| * erfindungsgemäße Verbindung | | |

Mit der erfindungsgemäßen Verbindung (1) konnte eine gegenüber dem Vergleichsliganden (2) gesteigerte Ausbeute erzielt werden.

Die durchgeführten Versuche belegen, dass die gestellte Aufgabe durch die erfindungsgemäße Verbindung gelöst wird.

## Patentansprüche

1. Verbindung gemäß der Struktur (**I**): 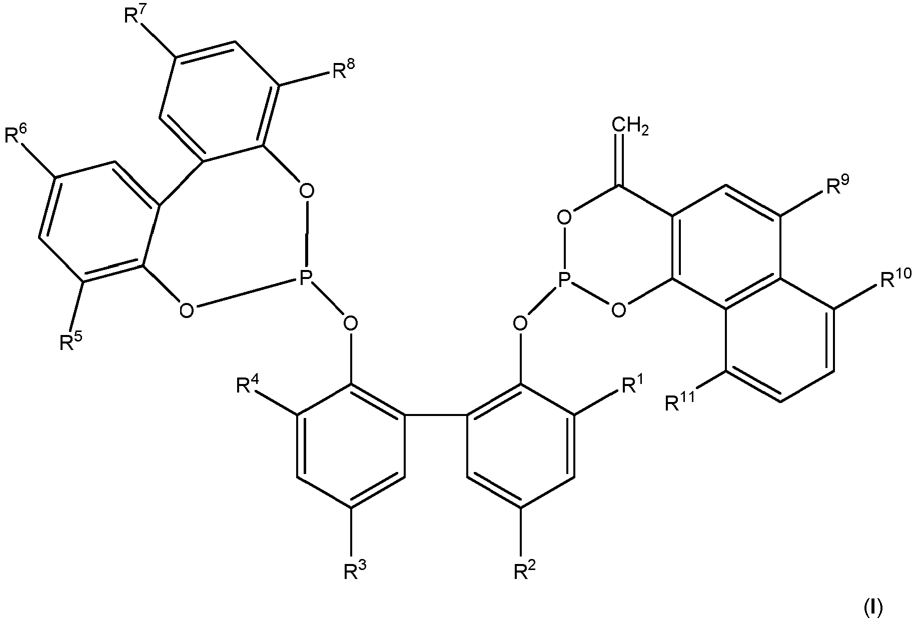 wobei R¹, R², R³, R⁴, R⁵, R⁶, R⁷, R⁸, R⁹, R¹⁰, R¹¹ ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -NO₂, -NH₂, -N[(C₁-C₁₂)-Alkyl]₂.

2. Verbindung nach Anspruch 1,
wobei R¹, R², R³, R⁴ ausgewählt sind aus: -H, -(C₁-C₁₂)-Alkyl, -O-(C₁-C₁₂)-Alkyl.

3. Verbindung nach Anspruch 1 oder 2,
wobei R⁵, R⁶, R⁷, R⁸ ausgewählt sind aus: -H, -(C₁-C₁₂)-Alkyl, -O-(C₁-C₁₂)-Alkyl.

4. Verbindung nach einem der Ansprüche 1 bis 3,
wobei R⁹, R¹⁰, R¹¹ ausgewählt sind aus: -H, -(C₁-C₁₂)-Alkyl, -O-(C₁-C₁₂)-Alkyl.

5. Verbindung nach einem der Ansprüche 1 bis 4,
wobei R¹, R², R³, R⁴, R⁵, R⁶, R⁷, R⁸, R⁹, R¹⁰, R¹¹ ausgewählt sind aus: -H, -(C₁-C₁₂)-Alkyl, -O-(C₁-C₁₂)-Alkyl.

6. Verbindung nach einem der Ansprüche 1 bis 5, wobei die Verbindung die Struktur (1) aufweist:

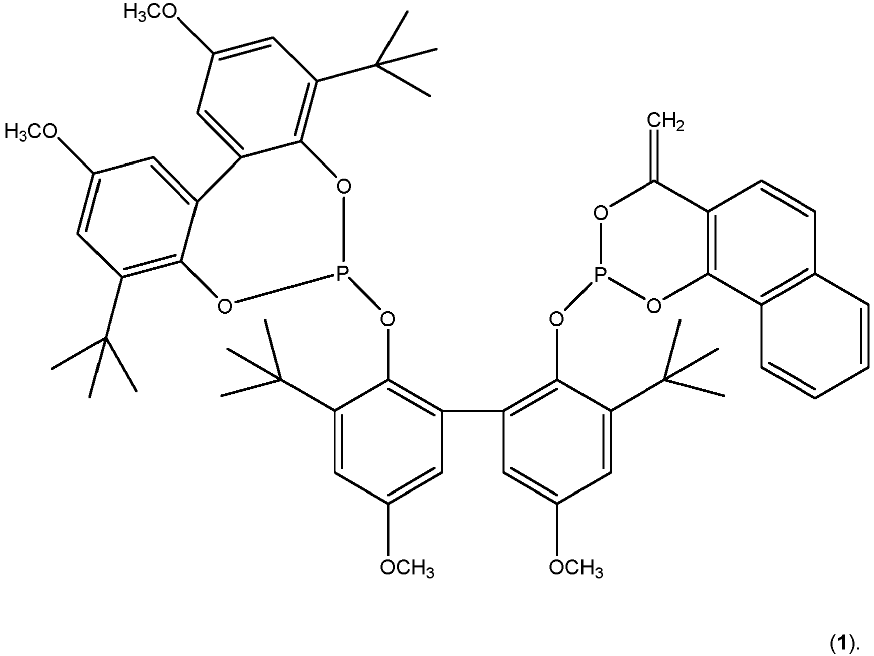

7. Verwendung einer Verbindung nach einem der Ansprüche 1 bis 6,
in einem Ligand-Metall-Komplex zur Katalyse einer Hydroformylierungsreaktion.

8. Verfahren umfassend die Verfahrensschritte:
a) Vorlegen eines Olefins,
b) Zugabe einer Verbindung nach einem der Ansprüche 1 bis 6 und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

## Claims

1. Compound according to the structure (I): 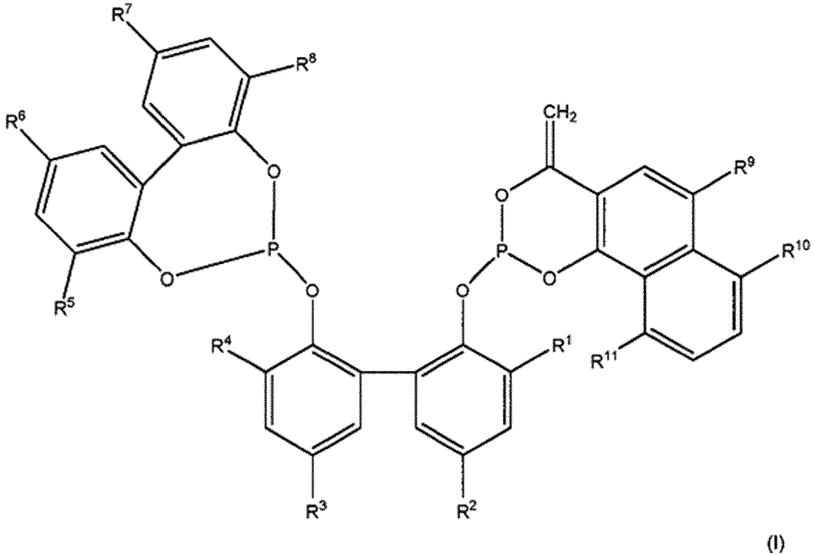 where R¹, R², R³, R⁴, R⁵, R⁶, R⁷, R⁸, R⁹, R¹⁰, R¹¹ are selected from: -H, -(C₁-C₁₂)-alkyl, -O-(C₁-C₁₂) -alkyl, -(C₆-C₂₀)-aryl, -O-(C₆-C₂₀)-aryl, -NO₂, NH₂, -N[((C₁-C₁₂) -alkyl)]₂.

2. Compound according to Claim 1,
where R¹, R², R³, R⁴ are selected from: -H, -(C₁-C₁₂) -alkyl, -O-(C₁-C₁₂) -alkyl.

3. Compound according to Claim 1 or 2,
where R⁵, R⁶, R⁷, R⁸ are selected from: -H, -(C₁-C₁₂) -alkyl, -O-(C₁-C₁₂) -alkyl.

4. Compound according to any of Claims 1 to 3,
where R⁹, R¹⁰, R¹¹ are selected from: -H, -(C₁-C₁₂) -alkyl, -O-(C₁-C₁₂) -alkyl.

5. Compound according to any of Claims 1 to 4,
where R¹, R², R³, R⁴, R⁵, R⁶, R⁷, R⁸, R⁹, R¹⁰, R¹¹ are selected from: -H, -(C₁-C₁₂) -alkyl, -O-(C₁-C₁₂)-alkyl

6. Compound according to any of Claims 1 to 5, where the compound has the structure (1):

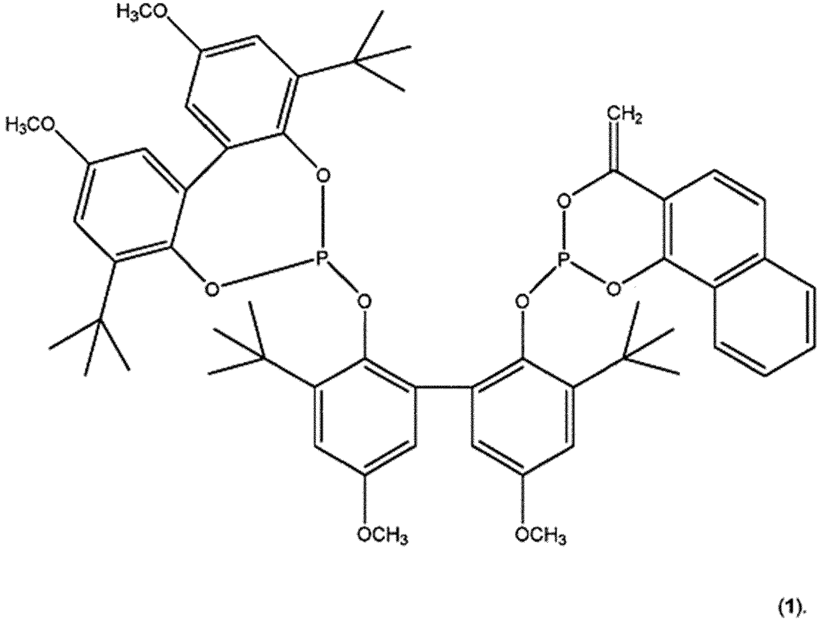

7. Use of a compound according to any of Claims 1 to 6 in a ligand-metal complex for catalysis of a hydroformylation reaction.

8. Process comprising the process steps of:
a) charging an olefin,
b) adding a compound according to any of Claims 1 to 6 and a substance comprising a metal selected from: Rh, Ru, Co, Ir,
c) feeding in H₂ and CO,
d) heating the reaction mixture of a) to c) wherein the olefin is converted to an aldehyde.

## Revendications

1. Composé selon la structure (I) : 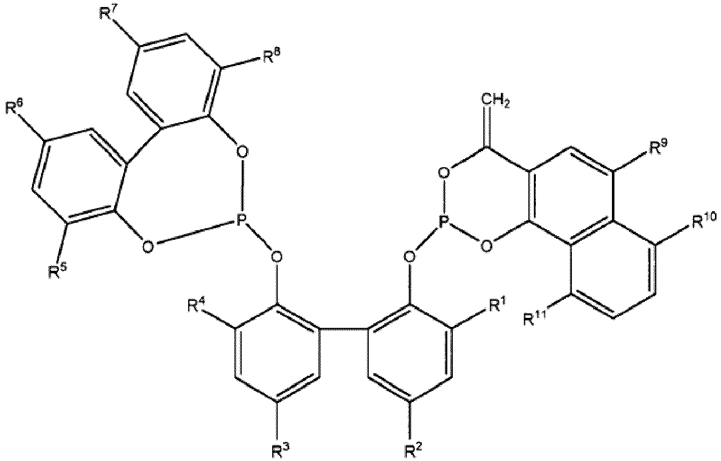 5 R¹, R², R³, R⁴, R⁵, R⁶, R⁷, R⁸, R⁹, R¹⁰, R¹¹ étant choisis parmi : -H, -(C₁-C₁₂) -alkyle, -O- (C₁-C₁₂) -alkyle, -(C₆-C₂₀) -aryle, -O-(C₆-C₂₀)-aryle, -NO₂, -NH₂, -N[(C₁-C₁₂) - alkyle]₂.

2. Composé selon la revendication 1,
R¹, R², R³, R⁴ étant choisis parmi : -H, -(C₁-C₁₂) -alkyle, -O-(C₁-C₁₂) -alkyle.

3. Composé selon la revendication 1 ou 2,
15 R⁵, R⁶, R⁷, R⁸ étant choisis parmi : -H, -(C₁-C₁₂) -alkyle, -O-(C₁-C₁₂) -alkyle.

4. Composé selon l'une quelconque des revendications 1 à 3,
R⁹, R¹⁰, R¹¹ étant choisis parmi : -H, -(C₁-C₁₂) -alkyle, - O-(C₁-C₁₂) -alkyle.

5. Composé selon l'une quelconque des revendications 1 à 4,
R¹, R², R³, R⁴, R⁵, R⁶, R⁷, R⁸, R⁹, R¹⁰, R¹¹ étant choisis parmi : -H, -(C₁-C₁₂) -alkyle, -O-(C₁-C₁₂) -alkyle.

6. Composé selon l'une quelconque des revendications 1 à 5, le composé présentant la structure (1) :

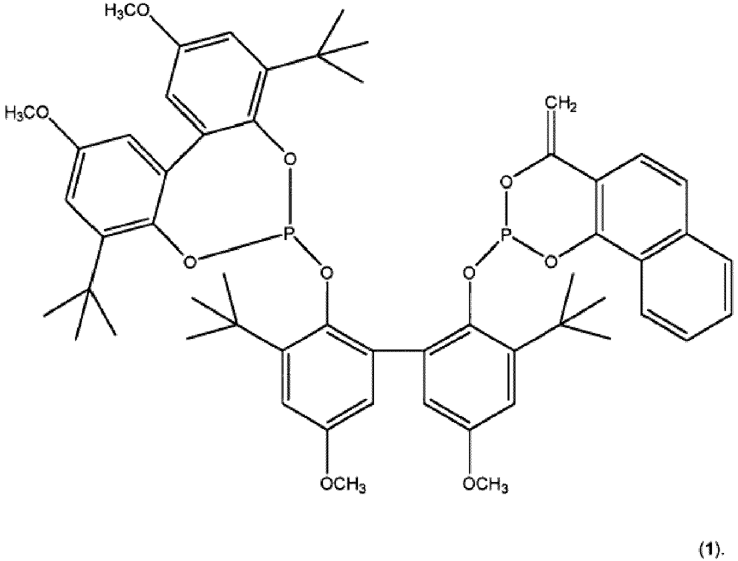

7. Utilisation d'un composé selon l'une quelconque des revendications 1 à 6, dans un complexe ligand-métal pour la catalyse d'une réaction d'hydroformylation.

8. Procédé comprenant les étapes de procédé de :
a) chargement d'une oléfine,
b) ajout d'un composé selon l'une quelconque des revendications 1 à 6 et d'une substance qui présente un métal choisi parmi : Rh, Ru, Co, Ir ;
c) introduction de H₂ et de CO,
d) chauffage du mélange réactionnel de a) à c), l'oléfine étant transformée en un aldéhyde.
